# EUROPEAN PATENT APPLICATION

(11) **EP 4 218 546 A1**
(43) Date of publication of application: **02.08.2023**
(21) Application number: 20955381.7
(22) Date of filing: 16.11.2020
(51) Int. Cl.: A61B 5/00, A61B 10/00, G16H 50/20

(54) **BREAST CANCER DIAGNOSTIC SYSTEM**

(30) Priority: 25.09.2020 KR 20200124448
(71) Applicant: Olive Healthcare Inc., Seoul 05836 (KR)
(72) Inventor: HAN, Sung Ho, Seoul 04732 (KR); PARK, Hyon Soo, Seoul 06999 (KR); HONG, Hee Sun, Gwacheon-si Gyeonggi-do 13818 (KR); CHOI, Suk Jin, Seoul 06611 (KR); BAN, Soon Hyun, Seoul 07633 (KR)
(74) Representative: Manitz Finsterwald Patent- und Rechtsanwaltspartnerschaft mbB
(86) International application number: PCT/KR2020/016091
(87) International publication number: WO 2022/065583

(57) **Abstract**

A breast cancer diagnosis system includes a probe that sequentially outputs light of a plurality of wavelengths of a near-infrared area to a target object, receives reflected light, and sequentially processes the reflected light; a central control device that receives optical data of the reflected light sensed by the probe, calculates a concentration of chromophore of the target object for each chromophore, and generates an image of the chromophores indicating a distribution of the concentration value of each chromophore; and a display that outputs the image, in which in the probe, one or more channel signal processing units including one or more light irradiation modules and light collection modules may be disposed, and each of the channel signal processing units sequentially operates so that each of the channel signal processing units may generate the optical data of the reflected light.

## Description

### BACKGROUND

### 1. Field

The present disclosure relates to a breast cancer diagnosis system, and more particularly, to a breast cancer diagnosis system for diagnosing breast cancer using near-infrared ray measurement technology.

### 2. Description of the Related Art

Early diagnosis and treatment of disease is important for leading a healthy life. Among various diseases, cancer is a serious life-threatening disease, and interest in early diagnosis and treatment of cancer is increasing.

On the other hand, in the case of women, the incidence of breast cancer appears to be the highest among cancers such as breast cancer, thyroid cancer, stomach cancer, colon cancer, and lung cancer. Therefore, in order to effectively treat breast cancer, attention has been focused on finding a method for effectively diagnosing breast cancer at an early stage.

As a conventional technology for diagnosing breast cancer, mammography is known to detect lesions present inside the breast using X-rays. However, the mammography has a problem in that it is difficult to select cancer from breast tissue because the difference in X-ray absorptivity between the breast tissue and cancer is very small.

As a way to solve this problem, there is a method for diagnosing breast cancer using diffuse optical tomography that obtains a two-dimensional or three-dimensional image of the tissue inside the breast using a wavelength of light.

For example, Korean Patent Unexamined Publication No. 10-2019-0048249 discloses a device and method for diagnosing breast cancer using the diffuse optical tomography.

Specifically, Korean Patent Unexamined Publication No. 10-2019-0048249 discloses a device that converts an optical signal and a signal obtained by detecting infrared rays irradiated onto a diagnosis target using the optical signal into digital signals, respectively, and a difference in signal size and phase difference between respective converted digital signals are calculated to diagnose breast cancer.

### SUMMARY

One object of the present disclosure is to provide a breast cancer diagnosis system capable of accurately diagnosing breast cancer while performing video recording more conveniently in a system for diagnosing breast cancer using Discrete Multi Wavelength Near Infra-Red Spectroscopy (DMW-NIRS).

The object of the present disclosure is not limited to the tasks mentioned above, and other tasks not mentioned will be clearly understood by those skilled in the art from the description below.

As technical means for achieve the technical object, a breast cancer diagnosis system according to an example of the present disclosure may be a breast cancer diagnosis system using near-infrared measurement technology, including: a probe that sequentially outputs light of a plurality of wavelengths of a near-infrared area to a target object, receives reflected light reflected from the target object, and sequentially processes the reflected light; a central control device that controls an operation of the probe, receives optical data of the reflected light sensed by the probe, calculates a concentration of chromophore of the target object for each chromophore based on the optical data, and generates an image of the chromophores indicating a distribution of the concentration value of each chromophore; and a display that outputs the image generated by the central control device, in which in the probe, one or more channel signal processing units including one or more light irradiation modules and one or more light collection modules may be disposed, and each of the channel signal processing units sequentially operates so that each of the channel signal processing units may generate the optical data of the reflected light.

In addition, the probe may include a body that includes the plurality of channel signal processing units, a first control unit controlling an operation of the channel signal processing unit, and a first communication unit transmitting output data of the first control unit to the outside; and a contact surface that is disposed on a lower surface of the body, the light irradiation module and the light collection module of the channel signal processing unit may be disposed in a state of being exposed to the outside through an opening formed on the contact surface, the one or more channel signal processing units may be disposed in a horizontal direction along one axis of the probe, and the light irradiation modules and the light collection modules of the channel signal processing unit adjacent to each other may be disposed adjacent to each other, the light irradiation modules may be disposed in a row parallel to the one axis, and the light collection modules may be disposed in a row parallel to the one axis by being spaced apart from the light irradiation modules by predetermined distances.

In addition, each of the channel signal processing units may include one or more light irradiation modules disposed adjacent to each other; the light collection module disposed by being spaced apart from the light irradiation module by predetermined distance; and a driving chip that sequentially outputs a driving signal for driving the light irradiation module according to a control signal of the first control unit and transmits the optical data of the reflected light detected by the light collection module to the first control unit, and the driving chip of each of the channel signal processing units may sequentially output 4 to 12 driving signals so that light of 4 to 12 different wavelengths may be sequentially output from the light irradiation module.

In addition, the first control unit may include a first decoder that sequentially transmits a control signal for operating the driving chip of each of the channel signal processing units; and a second decoder that sequentially receives and outputting the optical data of the reflected light output from each of the driving chips.

In addition, the probe may further include a blinking LED or liquid crystal display in a housing outside the body, and upon receiving all of the optical data of the reflected light corresponding to a unit scan through each of the channel signal processing units, the first control unit may display that the unit scan is completed through the blinking LED or the liquid crystal display.

In addition, the central control unit may calculate the concentration of the chromophore of the target object by inputting a measurement value for each wavelength of the reflected light sensed through the probe to a learning model machine-learned based on training data in which a measurement value for each wavelength of light and the concentration of each of a plurality of chromophore materials are matched respectively.

In addition, the central control device may sequentially receive unit scan data transmitted from each of the channel signal processing units, apply each of the unit scan data to the learning model to calculate the concentration of the chromophore, and display each concentration of the chromophore on a two-dimensional coordinates to generate a unit frame image is generated, the unit scan data may include the optical data of the reflected light reflected based on light of 4 or more and 12 or less different wavelengths output from the light irradiation module of each of the channel signal processing units, and data of each coordinate and an empty space between the coordinates may display an estimated value through an interpolation algorithm.

In addition, the central control unit may output an examination interface for guiding a scanning procedure through the probe, the diagnosis interface may include an imaging guide interface displaying a left chest area and a right chest area, respectively, and a first ROI interface displaying an imaging area requiring diagnosis and a second ROI interface displaying an area requiring imaging as a contrast area for the imaging area requiring the diagnosis may be displayed on the imaging guide interface.

In addition, the diagnosis interface may further include a scanning guide interface indicating a state of securing the unit scan data of the probe.

In addition, the central control device may output a diagnosis interface that outputs the chromophore image indicating a distribution state of the chromophores, and the diagnosis interface may dispose and display the chromophore image of a diagnosis area generated based on data collected using the first ROI interface and the chromophore image of a contrast area generated based on data collected using the second ROI interface, side by side.

In addition, the diagnosis interface may dispose and display the chromophore image of the diagnosis area and the chromophore image of the contrast area side by side for each type of the chromophore.

In addition, a breast cancer diagnosis method according to another example of the present disclosure may be a breast cancer diagnosis method using a breast cancer diagnosis system based on near-infrared measurement technology, the breast cancer diagnosis method including: receiving optical data of reflected light from a target object sensed by a probe; calculating a concentration of chromophore of the target object for each chromophore based on the optical data; generating an image of the chromophore indicating a distribution of the calculated concentration values of the chromophores; and outputting the image of the generated chromophore through a display, in which in the step of calculating the concentration of the chromophore, the concentration of the chromophore of the target object may be calculated by inputting a measurement value for each wavelength of the reflected light sensed through the probe to a learning model machine-learned based on training data in which a measurement value for each wavelength of light and the concentration of each of a plurality of chromophore materials are matched respectively.

Details of other examples for solving the problem are included in the description and drawings of the present disclosure

According to the above-described problem solving means of the present disclosure, in the breast cancer diagnosis system according to the present disclosure, the probe has a wireless structure, includes a plurality of channel signal processing units, and collects the optical data of a wide area of the breast at once so that it is possible to provide an effect of conveniently imaging the breast.

In addition, since an examination interface capable of accurately capturing the chest with a probe is output to the display under the control of the central control unit, an effect of accurately capturing the chest is provided.

In addition, since the diagnosis interface capable of accurately imaging the chest with a probe is output to the display under the control of the central control device, an effect of accurately imaging the chest is provided.

In addition, since the diagnosis interface to which the chromophore image of the diagnosis area and the chromophore image of the contrast area are output for each type of the chromophore is output to the display under the control of the central control device, an effect of accurately diagnosing breast cancer is provided by comparing the chromophore image of the diagnosis area and the chromophore image of the contrast area for each type of the chromophore.

### BRIEF DESCRIPTION OF THE DRAWINGS

FIG. 1 is a block diagram showing a breast cancer diagnosis system according to an example of the present disclosure;
FIG. 2 is a diagram showing an example of a probe of FIG. 1;
FIG. 3 is a diagram showing the probe of FIG. 2 collecting optical data by coming into contact with a target object;
FIG. 4 is a diagram showing another example of the probe of FIG. 1;
FIG. 5 is a diagram showing a first decoder and a second decoder of a first control unit that transmit and receive data to and from a driving chip of a channel signal processing unit;
FIG. 6 is a block diagram showing a data processing unit of a central control device of FIG. 1;
FIG. 7 is a block diagram showing an interface output to a display unit of a display under the control of a second control unit of the central control device of FIG. 1;
FIG. 8 is a block diagram showing an examination interface of FIG. 7;
FIG. 9 is a diagram showing an example of outputting the examination interface of FIG. 8 to a display unit of a display;
FIG. 10A and FIG. 10B are diagrams showing a scanning guide interface of FIG. 8 where the completion of collection of optical data for each channel signal processing unit is displayed;
FIG. 11A, FIG. 11B and FIG. 11C are diagrams showing the scanning guide interface of FIG. 8 where movement guidance of the probe for collecting optical data is displayed;
FIG. 12 is a diagram showing the diagnosis interface of FIG. 7 outputting a chromophore image;
FIG. 13 is a diagram showing the diagnosis interface of FIG. 7 where the chromophore image of a diagnosis area and the chromophore image of a contrast area are output for each type of the chromophore; and
FIG. 14 is a diagram showing another example in which an examination interface is output to a display unit of a display.

### DETAILED DESCRIPTION

Hereinafter, examples of the present application will be described in detail so that those skilled in the art may easily practice with reference to the accompanying drawings. However, the present disclosure may be implemented in many different forms and is not limited to the examples described herein. And in order to clearly describe the present application in the drawings, parts irrelevant to the description are omitted, and similar reference numerals are attached to similar parts throughout the specification.

Throughout this specification, when a part is said to be "connected" to another part, this includes not only the case of being "directly connected" but also the case of being "electrically connected" with another element therebetween.

Throughout the present specification, when a member is said to be positioned on another member, this includes not only the case where a certain member is in contact with another member, but also the case where another member exists between the two members.

Throughout the present specification, when a part "includes" a certain component, it means that it may further include other components without excluding other components unless otherwise stated. As used throughout this specification, the terms "about," "substantially," and the like are used at or approximating that value when manufacturing and material tolerances inherent in the stated meaning are given, and do not convey the understanding of this application. Accurate or absolute figures are used to help prevent exploitation by unscrupulous infringers of the disclosed disclosure. The term "step of (doing)" or "step of' as used throughout the present specification does not mean "step for".

Hereinafter, preferred examples of the present disclosure will be described in detail with reference to the accompanying drawings and the description below. However, the present disclosure is not limited to the examples described herein and may be embodied in other forms. Like reference numbers indicate like elements throughout the specification.

Hereinafter, a configuration of a breast cancer diagnosis system according to an example of the present disclosure will be described.

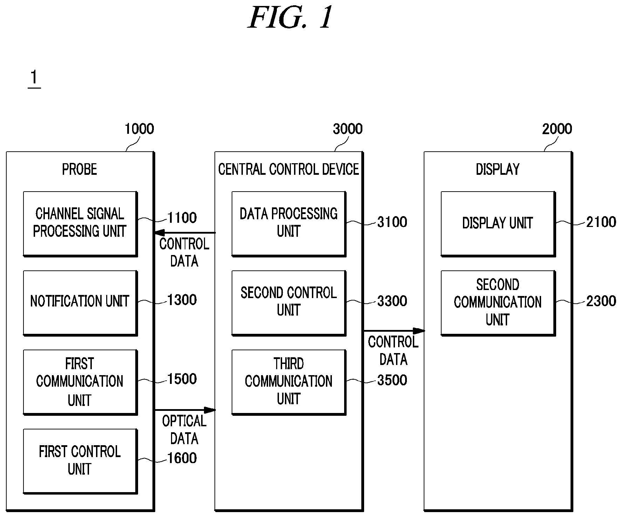

FIG. 1 is a block diagram showing a breast cancer diagnosis system according to an example of the present disclosure.

Referring to FIG. 1, a breast cancer diagnosis system 1 of the present disclosure includes a probe 1000 for collecting optical data for each chromophore material of breast tissue, a display 2000 for outputting an interface and an image, and a central control device 3000 that controls the operation of the probe 1000 and the output of the display 2000, and processes data.

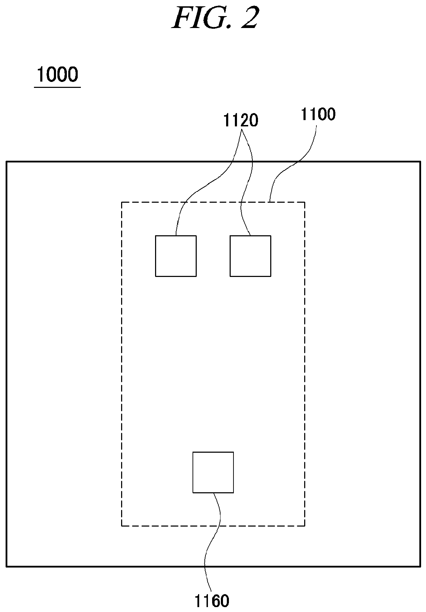

FIG. 2 is a diagram showing an example of the probe of FIG. 1.

First, a configuration of the probe 1000 will be described with reference to FIGS. 1 and 2.

The probe 1000 may include a channel signal processing unit 1100, a notification unit 1300, a first communication unit 1500, a first control unit 1600, and a body.

N (N is a natural number of 1 or more) channel signal processing units 1100 may be arranged in the probe 1000. Each of the channel signal processing units includes a light irradiation module 1120 for irradiating a breast with the incident light, a light collection module 1160 for collecting measurement values (hereinafter referred to as optical data) of information such as a wavelength of reflected light of the incident light irradiated to the breast (hereinafter referred to as the reflected light), and a driving chip.

The light irradiation module 1120 may be configured to include an optical output device such as a laser diode (LD), a light emitting diode (LED), or a vertical cavity surface emitting laser (VCSEL), capable of outputting incident light having wavelengths of different lengths in the near infrared ray area.

For example, the light irradiation module 1120 may be configured to output incident light classified into 8 types according to the length of the wavelength. In this case, if one light output device constituting the light irradiation module 1120 outputs light having 8 types of wavelengths, the light irradiation module 1120 may be configured of one light output device. In addition, if one light output device constituting the light irradiation module 1120 outputs light having 4 types of wavelengths, the light irradiation module 1120 may be configured of two light output devices each outputting light of different wavelengths.

On the other hand, the number of types of incident light for each wavelength output by the light irradiation module 1120 may be determined based on the number of types of the chromophores present in the breast. Since the chromophores effectively absorb light having a wavelength of a specific length depending on their type, incident light having a wavelength that is effectively absorbed for each chromophore is irradiated to the breast and the reflected light is collected and analyzed, and thereby the concentration of each type of the chromophore may be measured.

The light collection module 1160 may be configured to include a light collection device such as a photo diode, a photo transistor, a photo multiplier tube (PMT), or a photo cell capable of receiving light and collecting light data.

Optical data of the reflected light collected by the light collection module 1160 may be transmitted to the central control device 3000 to calculate the concentration of the chromophore.

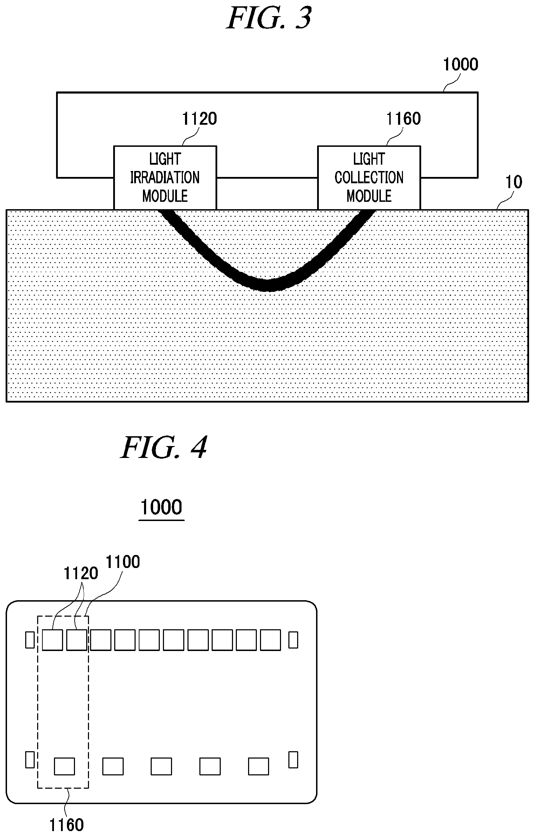

FIG. 3 is a diagram showing the probe of FIG. 2 collecting optical data by coming into contact with a target object.

Specifically, referring to FIG. 3, the light irradiation module 1120 and the light collection module 1160 are installed on the probe 1000 so as to be exposed to a predetermined surface of the probe 1000, and when the predetermined surface of the probe 1000 comes into contact with a light data collection target (hereinafter referred to as the target object) 10 such as the breast, the light irradiation module 1120 and the light collection module 1160 may come into contact with the target object 10.

Then, when the incident light irradiated from the light irradiation module 1120 is reflected inside the target object 10, the light collection module 1160 may collect optical data of the reflected light. The optical data may include information about the wavelength and intensity of the reflected light, and the like.

FIG. 4 is a diagram showing another example of the probe of FIG. 1.

Referring to FIG. 4, the probe 1000 may include one or more channel signal processing units 1100.

For example, as shown in FIG. 4, a plurality of channel signal processing units 1100 may be included in the probe 1000 so that a plurality of light irradiation modules 1120 and a plurality of light collection modules 1160 are exposed to the predetermined surface of the probe 1000.

For example, N channel signal processing units 1100 may be disposed in a horizontal direction along one axis of the probe 1000, the light irradiation modules 1120 and the light collection module 1160 of the channel signal processing unit 1100 adjacent to each other may be disposed so as to be adjacent to each other, so that the light irradiation modules 1120 may be disposed in a row parallel to one axis thereof, and the light collection modules 1160 may be disposed in a row parallel to one axis thereof by being spaced apart from the light irradiation modules 1120 by predetermined distances. In this case, a penetration depth of the near-infrared rays may be adjusted by the separation distance between the light collection module 1160 and the light irradiation module 1120, and accordingly, a measurement depth in the measurement target may be adjusted. For example, the penetration depth of near-infrared rays is about 1/2 of the separation distance. Accordingly, an optimal measurement depth may be set by setting an appropriate separation distance between the light collection module 1160 and the light irradiation module 1120.

In other words, the distances between the light irradiation modules 1120 and the light collection modules 1160 included in each channel signal processing unit 1100 are the same, so that the plurality of light irradiation modules 1120 may be disposed in a row in a first direction on the predetermined surface, and the plurality of light collection modules 1160 may be disposed in a row in a second direction parallel to the first direction on the predetermined surface of the probe 1000.

As such, since the probe 1000 includes one or more channel signal processing units 1100, when collecting the optical data by contacting the target object 10, the probe 1000 may collect the optical data covering a wide area of the target object 10 with one contact.

On the other hand, a disposition structure of the plurality of light irradiation modules 1120 and the light collection modules 1160 included in the plurality of channel signal processing units 1100 installed in the probe 1000 is not limited to the above-described disposition structure of light irradiation modules 1120 and the light collection modules 1160.

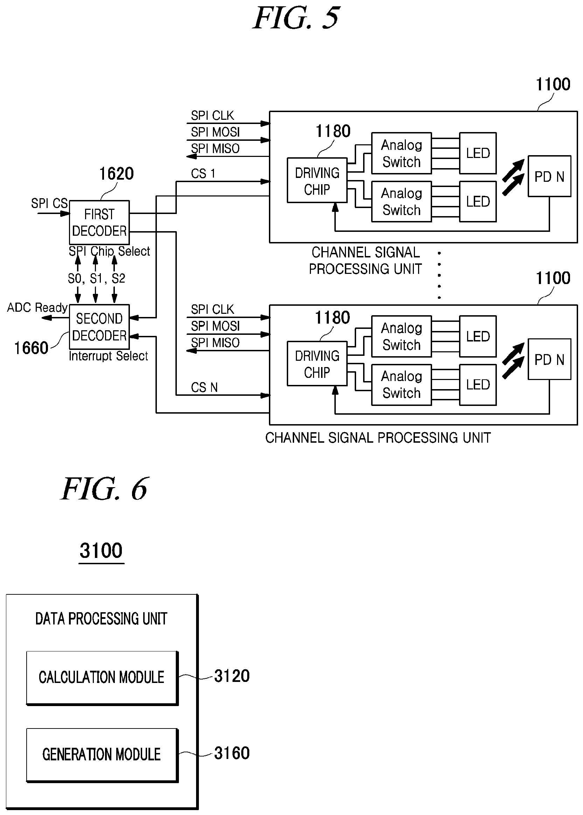

A driving chip 1180 in FIG. 5 may sequentially output a driving signal for driving the light irradiation module 1120 according to a control signal of the first control unit 1600 which is described later, and transmit the optical data of the reflected light detected by the light collection module 1160 to the first control unit 1600.

In addition, the driving chip 1180 of each channel signal processing unit 1100 may sequentially output 4 to 12 driving signals so that light of 4 to 12 different wavelengths may be sequentially output from the light irradiation module 1120.

The notification unit 1300 may be installed on a housing outside the body of the probe 1000, including a flickering LED and a liquid crystal display, and may perform a function of notifying a user of an operating state of the probe 1000.

For example, when the channel signal processing unit 1100 of the probe 1000 comes into contact with the breast and completes the collection of the optical data, the notification unit 1300 blinks the blinking LED or displays the information about the completion of the collection of the optical data on the liquid crystal display, so that the completion of the optical data collection of the probe 1000 may be notified to the user.

The first communication unit 1500 may be configured of a conventional communication module capable of transmitting and receiving data with a device outside the probe 1000, and may be configured to include a wireless communication module such as Wi-Fi or Bluetooth^{®}.

For example, in a case where the probe 1000 is configured to be wirelessly connected to the central control device 3000, control data related to the operation of the probe 1000 transmitted from the central control device 3000 may be received through the first communication unit 1500. In addition, the optical data of the breast collected by the probe 1000 may be transmitted to the central control device 3000 through the first communication unit 1500.

The first control unit 1600 may control the operations of the channel signal processing unit 1100, the notification unit 1300, and the first communication unit 1500, and may be implemented as a device capable of processing data such as a processor. Here, the processor may mean, for example, a data processing device built in hardware having a physically structured circuit to perform functions expressed by codes or instructions included in a program. An example of such a data processing device built in hardware may include a microprocessor, a central processing unit (CPU), a processor core, a multiprocessor, an application-specific integrated circuit (ASIC), a field programmable gate array (FPGA), or the like, but the scope of the present disclosure is not limited thereto.

In addition, the first control unit 1600 may further include a first decoder 1620 and a second decoder 1660 that transmit and receive data to and from the driving chip 1180 of the channel signal processing unit 1100.

FIG. 5 is a diagram showing the first decoder and the second decoder of the first control unit that transmit and receive data to and from the driving chip 1180 of the channel signal processing unit.

On the other hand, referring to FIG. 5, the first control unit 1600 may include the first decoder 1620 that sequentially transfers control signals for operating the driving chips 1180 of each channel signal processing unit and the second decoder 1660 that sequentially receives and outputs the optical data of the reflected light output from each driving chip 1180.

In addition, as receiving all the optical data of the reflected light corresponding to the unit scan which is described later through each channel signal processing unit 1100, the first control unit 1600 may transmit the control signal to cause the notification unit 1300 such as a blinking LED or a liquid crystal display to display the completion of the unit scan.

The body may be configured of a housing capable of accommodating the channel signal processing unit 1100, the notification unit 1300, the first communication unit 1500, and the first control unit 1600 therein, and a contact surface thereof may be disposed on a lower surface. The light irradiation module 1120 and the light collection module 1160 may be disposed to be exposed to the outside through an opening formed on the contact surface.

Next, the configuration of the display 2000 will be described.

Referring to FIG. 1, the display 2000 may include a display unit 2100 and a second communication unit 2300.

The display unit 2100 may be configured of a conventional monitor, liquid crystal display, or the like capable of outputting various image data as images or outputting an interface.

The second communication unit 2300 may be configured of a conventional communication module capable of transmitting and receiving data to and from a device outside the display 2000.

For example, in a case where the display 2000 is configured to be wirelessly connected to the central control device 3000, the image data or the like transmitted from the central control device 3000 may be received through the second communication unit 2300.

In addition, the display 2000 may further include a touch input module, and accordingly, operations of various interfaces displayed on the display 2000 may be controlled, and operation control of the central control device 3000 may be performed therethrough.

Next, the configuration of the central control device 3000 will be described.

Referring to FIG. 1, the central control device 3000 may include a data processing unit 3100 that calculates and processes data, a second control unit 3300 that controls the operations of the probe 1000 and a display 2000, and a third communication unit 3500.

The central control device 3000 performs operations of controlling the operation of the probe, receiving the optical data of the reflected light sensed by the probe, calculating the concentration of the chromophore of the target object for each chromophore based on the optical data, and generating the image of the chromophore indicating the distribution of concentration values of the chromophore, and may include a memory in which a program for performing such operations built, and various processors for executing the program.

The memory may perform a function of temporarily or permanently storing data processed by the processor. Here, the memory may include volatile storage media or non-volatile storage media, but the scope of the present disclosure is not limited thereto.

The processor executes a program, and may refer to, for example, a data processing device built in hardware having a physically structured circuit to perform functions expressed by codes or instructions included in the program. An example of such a data processing device built in hardware may include all processing devices such as a microprocessor, a central processing unit (CPU), a processor core, a multiprocessor, an application-specific integrated circuit (ASIC), and a field programmable gate array (FPGA), but the scope of the present disclosure is not limited thereto.

FIG. 6 is a block diagram showing the data processing unit of the central control device of FIG. 1.

Referring to FIG. 6, the data processing unit 3100 includes a calculation module 3120 that calculates data on the concentration of the chromophore based on the optical data transmitted from the probe 1000, and a generation module 3160 that generates an image of the chromophore indicating the distribution of concentration values of the chromophores based on the calculated concentration of the chromophore.

The calculation module 3120 may receive the optical data (hereinafter, referred to as unit scan data) collected by each channel signal processing unit 1100 and calculate the concentration of the chromophore based on each unit scan data. The unit scan data may include measurement values of the reflected light reflected based on a plurality of different wavelengths of light output from the light irradiation module 1120 of each channel signal processing unit 1100.

For example, the calculation module 3120 may calculate the concentration of the chromophore using a machine-learned learning model (hereinafter, referred to as a learning model).

The learning model may be learned based on training data including information about incident light having a wavelength of a specific length, information about the optical data of the reflected light collected as the incident light having a wavelength of a specific length is irradiated to the breast, and information about the concentration of the chromophore corresponding to the information about the collected optical data.

Specifically, the training data is obtained by matching a measurement value for each wavelength of the reflected light with the concentration of each of a plurality of chromophore materials. For example, in a case where the incident light of the first to eighth wavelengths is sequentially incident, measurement values of the reflected light (for example, intensity of light) are measured from a1 to a8, and concentrations b1 to bn of individual chromophore material may be calculated based on the measurement values at this time, according to a known chromophore calculation equation. In this case, since the method for calculating the concentration of the chromophore included in a turbid medium by measuring the absorption coefficient and the scattering coefficient of the turbid medium in the near-infrared area corresponds to that of the prior art, a detailed description thereof will be omitted.

For example, in the present disclosure, when the concentrations of 4 chromophore materials such as water (H2O), lipid, oxy-hemoglobin (O2Hb), and deoxy-hemoglobin (HHb) are calculated based on 8 measurement values of reflected light, the concentrations b1, b2, b3, and b4 of each chromophore calculated for each of the measurement values a1 to a8 of the light data of the reflected light may be labeled and used as the learning data. The learning model may be built through a process of collecting a plurality of such learning data and applying them to a machine learning algorithm.

When specific optical data collected by irradiating the incident light having a specific wavelength is input to the machine-learned learning model based on such training data, the information about the input incident light and optical data may be compared with the corresponding training data to calculate the concentration of a specific chromophore of the breast.

On the other hand, the collected optical data may be input to the learning model after being calibrated by using a calibration value.

For example, the calibration value may be a value obtained by dividing a theoretical measurement value of the light data of the reflected light collected by irradiating the incident light on the target (hereinafter referred to as a reference target) that is a standard for measuring the calibration value with the light irradiation module 1120 by a measurement value of the optical data of the reflected light collected by actually irradiating the incident light on the reference target with the light irradiation module 1120.

In addition, the collected optical data may be calibrated by multiplying the measurement value of the optical data of the reflected light collected by the light collection module 1160 by irradiating incident light on the breast with the light irradiation module 1120 by the calibration value.

As described above, when the calibrated optical data is input to the learning model, the concentration of the chromophore may be calculated.

The generation module 3160 may generate the chromophore image including information about an image in which at least one of color, brightness, and chroma differs according to the concentration of the chromophore.

For example, the generation module 3160 may generate the chromophore image in which each concentration of the chromophore calculated based on each unit scan data may be displayed as an individual image (hereinafter referred to as a unit frame image) on two-dimensional coordinates.

In addition, the generation module 3160 may generate data about an image (hereinafter referred to as image data) using a conventional interpolation algorithm. As the interpolation algorithm, linear interpolation, bilinear interpolation, cubic interpolation, bicubic interpolation, or the like may be used.

For example, in a case where a chromophore image having a first chroma for a first position of the breast and a chromophore image having a second chroma for a second position of the breast are generated, estimated image data (hereinafter, referred to as estimated image data) may be generated by using the interpolation algorithm so that image data having an average chroma of the first and second chromas corresponds to an intermediate position of the first position and the second position.

The second control unit 3300 may control the operation of the probe 1000 by transmitting control data for controlling the operation of the probe 1000 to the probe 1000.

Specifically, the second control unit 3300 may control the operations of the light irradiation module 1120 of the channel signal processing unit 1100 and the notification unit 1300 of the probe 1000, and the probe 1000 may transmit optical data to the central control device 3000.

For example, the second control unit 3300 may control blinking of the incident light output from the light irradiation module 1120 so that the probe 1000 may irradiate the incident light to the breast or stop irradiating the incident light, and control the intensity and wavelength length of the incident light output from the light irradiation module 1120.

In addition, in a case where the probe 1000 includes a plurality of channel signal processing units 1100, the second control unit 3300 may control the irradiation order of the incident light so that the incident light may be irradiated in a free order or sequentially from the plurality of light irradiation modules 1120.

As another example, when the probe 1000 comes into contact with the breast and all of the plurality of channel signal processing units 1100 complete the collection of the optical data, the second control unit 3300 may control the operation of the notification unit 1300 to allow the notification unit 1300 to perform the operation of notifying completion of collection of the optical data.

As another example, the second control unit 3300 may control the probe 1000 to transmit the optical data collected by the probe 1000 to the central control device 3000 through the first communication unit 1500.

The second control unit 3300 may control the output of the display unit 2100 by transmitting the control data for controlling the operation of the display 2000 to the display 2000.

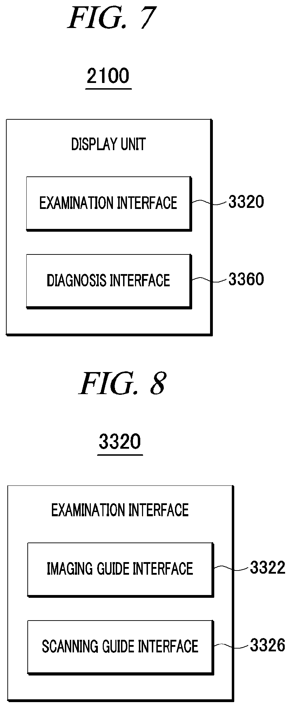

FIG. 7 is a block diagram showing an interface output to the display unit of the display under the control of the second control unit of the central control device of FIG. 1.

Referring to FIG. 7, the second control unit 3300 may control the display unit 2100 to output an interface including an examination interface 3320 for guiding examination of breast cancer and a diagnosis interface 3360 for diagnosing breast cancer to the display unit 2100.

FIG. 8 is a block diagram showing the examination interface of FIG. 7.

Referring to FIG. 8, the examination interface 3320 may include an imaging guide interface 3322 and a scanning guide interface 3326.

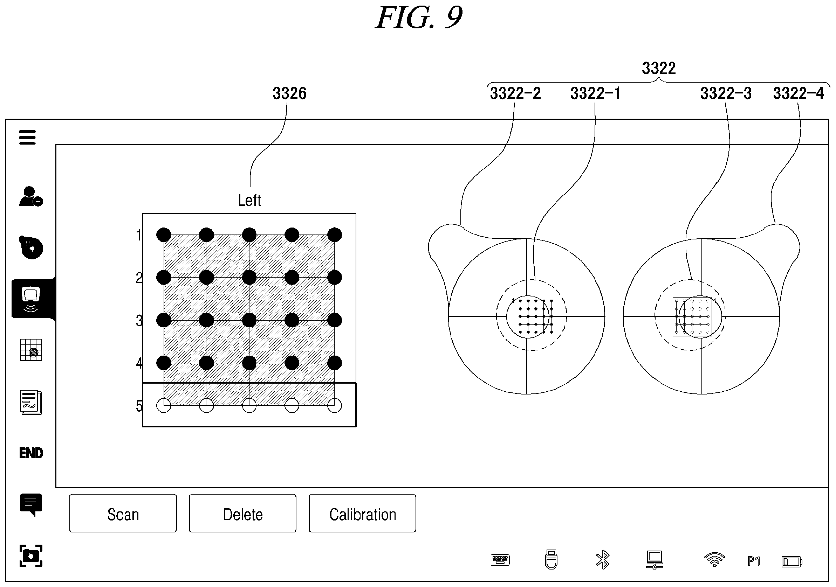

FIG. 9 is a diagram showing an example of outputting the examination interface of FIG. 8 to the display unit of the display.

Referring to FIG. 9, the imaging guide interface 3322 may display the left breast area and the right breast area, respectively.

Specifically, the imaging guide interface 3322 may include a first ROI interface 3322-1 in which a first portion to be examined for breast cancer is displayed in a first coordinate system, a first breast image 3322-2 including the first portion, a second ROI interface 3322-3 in which a second portion to be compared with the first portion is displayed in a second coordinate system, and a second breast image 3322-4 including the second portion.

The second breast image 3322-4 is an image for one breast separate from the first breast image 3322-2 among the subject's two breasts, and the second ROI interface 3322-3 may be a portion on the second breast which is symmetrical with the first ROI interface 3322-1 based on the center of the two breasts of the user. In this case, the first ROI interface indicates an area requiring scanning and may be directly set by the user of the device of the present disclosure. On the other hand, the second ROI interface is provided for scanning a contrast area to be compared, and may be directly set by the user or automatically set in a mirror-reflective area of the first ROI interface after the first ROI interface is set.

The scanning guide interface 3326 may be configured to guide the operation of the probe 1000 so that the user may collect the optical data inside the breast using the probe 1000, and may be configured of a third coordinate system in which the first portion 3322-1 or the second portion 3322-3 is enlarged and displayed.

Specifically, in a case where the user collects the optical data of the first breast image 3322-2 with the probe 1000, the scanning guide interface 3326 may include coordinates of a plurality of positions (hereinafter referred to as contact positions) at which a plurality of channel signal processing units 1100 come into contact with the first portion 3322-1, and coordinates of positions (hereinafter referred to as expected contact positions) at which the plurality of channel signal processing units 1100 are expected to come into contact with the first portion 3322-1.

In addition, in a case where the user collects the optical data of the second breast image 3322-4 with the probe 1000, the scanning guide interface 3326 may include coordinates of contact positions between the plurality of channel signal processing units 1100 and the second portion 3322-3, and coordinates of the expected contact positions between the plurality of channel signal processing units 1100 and the second portion 3322-3.

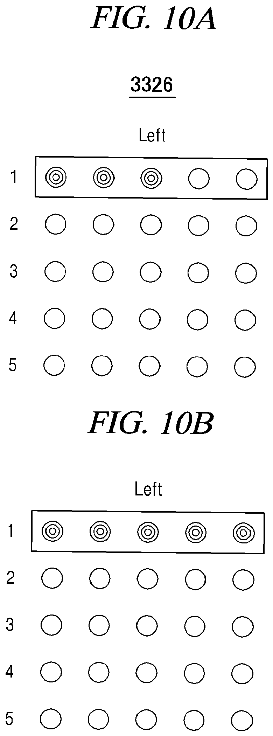

FIG. 10A and FIG. 10B are diagrams showing the scanning guide interface of FIG. 8 where the completion of collection of the optical data for each channel signal processing unit is displayed.

For example, referring to FIG. 10 A and FIG. 10B, in a case where the probe 1000 includes five channel signal processing units 1100, the five circular coordinates of the first row may be coordinates of contact positions between each channel signal processing unit 1100 of the probe 1000 and the first portion 3322-1 or the second portion 3322-3.

In addition, the circular coordinates of the second to fifth rows may be coordinates of the expected contact positions between each channel signal processing unit 1100 of the probe 1000 and the first portion 3322-1 or the second portion 3322-3.

On the other hand, when the individual channel signal processing unit 1100 completes collection of the optical data, the scanning guide interface 3326 may be controlled by the second control unit 3300 so that the completion of the collection of the optical data is displayed at a position on the third coordinate system corresponding to the contact position at which the collection of the optical data is completed. That is, it may indicate a state of securing the unit scan data of the probe 1000.

For example, referring to FIG. 10A, in a case where each channel signal processing unit 1100 of the probe 1000 coming into contact with the first portion 3322-1 or the second portion 3322-3 completes the detection of the optical data of the position of the first portion 3322-1 or the second portion 3322-3 corresponding to the circular coordinates relevant to the first to third columns of the first row, at positions corresponding to five circular coordinates in the first row, the second control unit 3300 may control the display unit 2100 so that the circular coordinates corresponding to the first to third columns of the first row are turned on.

As another example, referring to FIG. 10B, in a case where the channel signal processing unit 1100, which completes the detection of the optical data as shown in FIG 10A, completes the detection of the optical data of the position of the first portion 3322-1 or the second portion 3322-3 corresponding to the circular coordinates relevant to the fourth and fifth columns of the first row, the second control unit 3300 may control the display unit 2100 so that the circular coordinates corresponding to the fourth and fifth columns of the first row are turned on.

In addition, when the probe 1000 comes into contact with the first portion 3322-1 or the second portion 3322-3 so that the plurality of channel signal processing units 1100 constituting the probe 1000 complete the collection of all optical data, the scanning guide interface 3326 may be controlled by the second control unit 3300 to guide the movement of the probe 1000 so that the probe 1000 moves on the first portion 3322-1 or the second portion 3322-3 to collect the optical data.

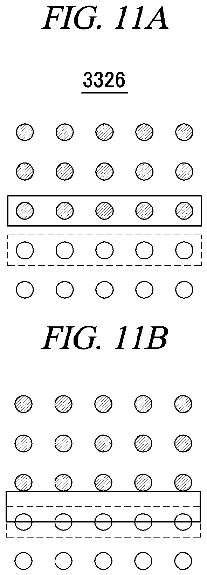

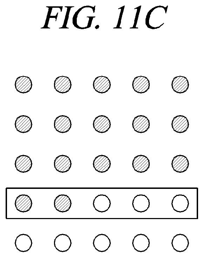

FIG. 11A, FIG. 11B and FIG. 11C are diagrams showing the scanning guide interface of FIG. 8 where the movement guidance of the probe for collecting the optical data is displayed.

For example, referring to FIG. 11A, the probe 1000 may be located such that five channel signal processing units 1100 are located at the positions of the first portion 3322-1 or the second portion 3322-3 corresponding to five circular coordinates of the third row. On the other hand, the position of the probe 1000 may be controlled by the second control unit 3300 to be displayed as a solid line on the third coordinate system.

In addition, in a case where the plurality of channel signal processing units 1100 of the probe 1000 coming into contact with the breast completes the detection of the optical data of the position of the first portion 3322-1 or the second portion 3322-3 corresponding to the circular coordinates relevant to the first to fifth columns of the third row, at the position of the first portion 3322-1 or the second portion 3322-3 corresponding to the five circular coordinates of the third row, the second control unit 3300 may control the display unit 2100 so that a dotted line guiding a position to move the probe 1000 is displayed around the fourth column.

Referring to FIG. 11B, as the probe 1000 is moved to the position guided by the dotted line, the solid line indicating the position of the probe 1000 may also move.

Next, referring to FIG. 11C, when the probe 1000 is moved such that the solid line indicating the position of the probe 1000 coincides with the dotted line displayed around the fourth column, the channel signal processing unit 1100 of the moved probe 1000 collects the optical data of the position of the first portion 3322-1 or the second portion 3322-3 corresponding to the circular coordinates of the fourth column.

On the other hand, the method for displaying the position of the probe 1000 and the method for guiding the position at which the probe 1000 is moved under the control of the second control unit 3300 are not limited to the aforementioned methods.

The diagnosis interface 3360 may include a chromophore image indicating a distribution state of the chromophores. That is, the diagnosis interface 3360 may output the chromophore image as an image to the display unit 2100 under the control of the second control unit 3300.

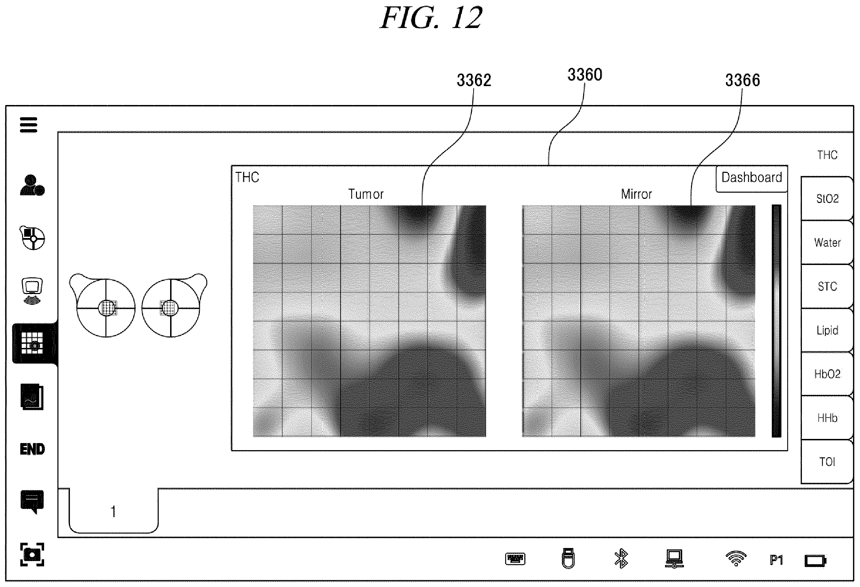

FIG. 12 is a diagram showing the diagnosis interface of FIG. 7 outputting the chromophore image.

Referring to FIG. 12, the diagnosis interface 3360 may be controlled by the second control unit 3300 so that the chromophore image 3362 of the diagnosis area which is the first area 3322-1 generated based on data collected by using the first ROI interface, and the chromophore image 3366 of the contrast area which is the second area 3322-3 generated based on the data collected by using the second ROI interface are simultaneously output.

In this way, when the chromophore image 3362 of the diagnosis area and the chromophore image 3366 of the contrast area are simultaneously output to the diagnosis interface 3360, the user may easily compare the two images and diagnose breast cancer quickly and accurately. In the drawing, the same image is output for convenience of description, but when cancer is present, the chromophore image 3362 of the diagnosis area and the chromophore image 3366 of the contrast area will be output differently.

The chromophore image 3362 of the diagnosis area and the chromophore image 3366 of the contrast area may include an estimated image, and the estimated image generated by the generation module 3160 of the second control unit 3300 may be output as an image to the display unit 2100.

For example, when the generation module 3160 generates the estimated image data using the above-described interpolation algorithm as the image data corresponding to some positions of the first portion 3322-1 and the second portion 3322-3 where the optical data is not collected, the second control unit 3300 may control the display unit 2100 so that the estimated image is output as an image corresponding to some positions of the first portion 3322-1 and the second portion 3322-3 where the optical data is not collected.

That is, the estimated image generated based on the estimated image data estimated through the interpolation algorithm may be displayed in an empty space between the coordinates.

In this way, an image reasonably estimated as an image of a position where the optical data is not collected is output to the chromophore image 3362 of the diagnosis area and the chromophore image 3366 of the contrast area output to the diagnosis interface 3360. Therefore, the user may be provided with continuous images of the concentration of the chromophore without interruption so as to facilitate diagnosis of breast cancer.

On the other hand, the diagnosis interface 3360 may be controlled by the second control unit 3300 to output the chromophore images for each type of the chromophore.

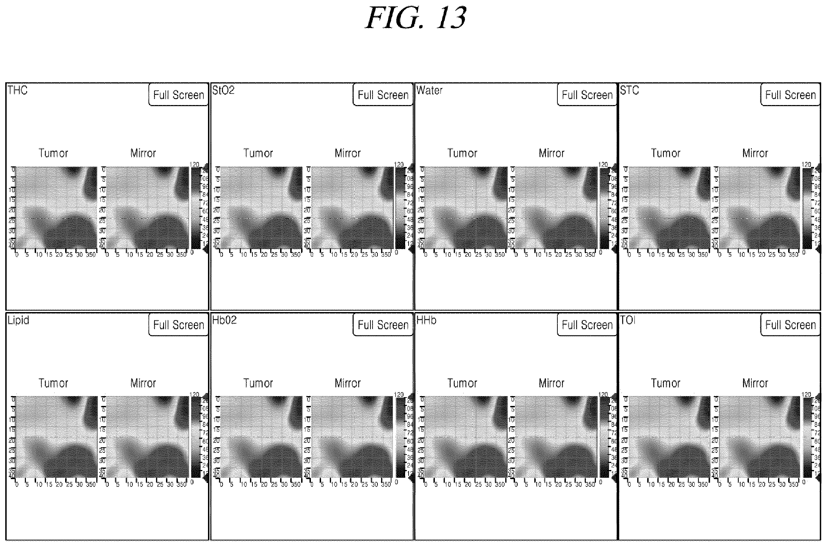

FIG. 13 is a diagram showing the diagnosis interface of FIG. 7 where the chromophore image of the diagnosis area and the chromophore image of the contrast area are output for each type of the chromophore.

For example, referring to FIG. 13, the diagnosis interface 3360 may be controlled by the second control unit 3300 to output the chromophore image of the diagnosis area and the chromophore image of the contrast area for each type of the chromophore.

In this way, when the chromophore image of the diagnosis area and the chromophore image of the contrast area are output to the diagnosis interface 3360 together for each type of the chromophore, the user may diagnose breast cancer more accurately by comparing in detail the chromophore image of the diagnosis area and the chromophore image of the contrast area for each type of chromophore.

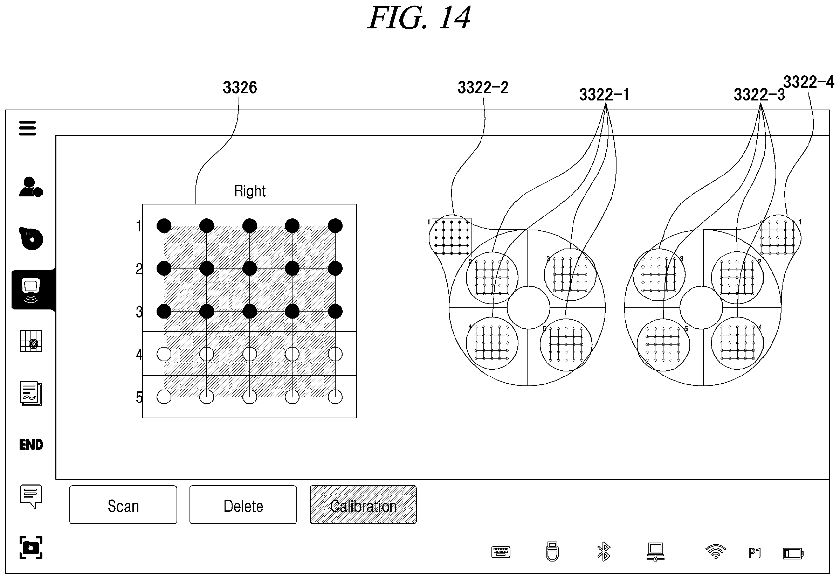

FIG. 14 is a diagram showing another example in which the examination interface is output to the display unit of the display.

The examination interface 3320 according to another example of the present disclosure has almost the same overall interface configuration as the example shown in FIG. 9. As shown in FIG. 14, in the present disclosure, a plurality of ROI interfaces 3322-1 and 3322-3 may be displayed on the breast image, and accordingly, the optical data may be collected for each of the ROI interfaces 3322-1 and 3322-3.

For example, four ROI interfaces 3322-1 may be set on the first breast image 3322-2 by the user, and accordingly, and four ROI interfaces 3322-3 may be set on the second breast image 3322-4 for collecting the optical data of the contrast area.

Hereinafter, the operation and effect of the breast cancer diagnosis system of the present disclosure will be described.

The user uses the probe 1000 according to the guidance of the examination interface 3320 output to the display unit 2100 of the display 2000 to collect the optical data of the first portion 3322-1 of the first breast image 3322-2 to examine breast cancer and the second portion 3322-3 of the second breast image 3322-4 to be compared thereto.

At this time, the optical data of the first portion 3322-1 and the second portion 3322-3 may be conveniently and effectively collected by moving the probe 1000 according to the guidance of the imaging guide interface 3322 and the scanning guide interface 3326 of the examination interface 3320.

The collected optical data of the first portion 3322-1 and the second portion 3322-3 is transmitted to the central control device 3000, and the central control device 3000 calculates the concentration of the chromophore based on the received optical data, and generates the chromophore image based on the calculated concentration of the chromophore.

When the chromophore image is generated, the central control device 3000 controls the display 2000 so that the diagnosis interface 3360 including the chromophore image of the diagnosis area and the chromophore image of the contrast area is output through the display unit 2100 of the display 2000.

The chromophore image of the diagnosis area and the chromophore image of the contrast area may be simultaneously output to the diagnosis interface 3360, and the user may diagnose breast cancer easily and quickly by simultaneously comparing the chromophore image of the diagnosis area and the chromophore image of the contrast area output to the diagnosis interface 3360.

In addition, since the chromophore image of the diagnosis area and the chromophore image of the contrast area may be output simultaneously to the diagnosis interface 3360 for each type of the chromophore, the user may accurately diagnose breast cancer by comparing and analyzing the chromophore image of the diagnosis area and the chromophore image of the contrast area for each type of the chromophore.

As described above, in the breast cancer diagnosis system according to the present disclosure, the probe has a wireless structure, includes a plurality of channel signal processing units, and collects the optical data of a wide area of the breast at once so that it is possible to provide an effect of conveniently imaging the breast.

In addition, since the examination interface capable of accurately imaging the breast with a probe is output to the display under the control of the central control device, an effect of accurately imaging the breast is provided.

In addition, since the chromophore image of the diagnosis area and the chromophore image of the contrast area are simultaneously output to the display under the control of the central control device, an effect of easily identifying breast cancer is provided by simultaneously comparing the chromophore image of the diagnosis area and the chromophore image of the contrast area.

In addition, since the diagnosis interface to which the chromophore image of the diagnosis area and the chromophore image of the contrast area are output for each type of the chromophore is output to the display under the control of the central control device, an effect of accurately diagnosing breast cancer is provided by comparing the chromophore image of the diagnosis area and the chromophore image of the contrast area for each type of the chromophore.

The method for diagnosing breast cancer according to an example of the present disclosure may be implemented in a form of a recording medium including instructions executable by a computer, such as a program module executed by a computer. Computer readable media may be any available media that may be accessed by a computer and includes both volatile and nonvolatile media, removable and non-removable media. In addition, computer readable media may include computer storage media. Computer storage media includes both volatile and nonvolatile, removable and non-removable media implemented in any method or technology for storage of information such as computer readable instructions, data structures, program modules or other data. Although the methods and systems of the present disclosure have been described with reference to specific examples, some or all of their components or operations may be implemented using a computer system having a general-purpose hardware architecture.

The above description of the present disclosure is for illustrative purposes, and those skilled in the art may understand that it may be easily modified into other specific forms without changing the technical spirit or essential features of the present disclosure. Therefore, the examples described above should be understood as illustrative in all respects and not limiting. For example, each component described as a single type may be implemented in a distributed manner, and similarly, components described as distributed may be implemented in a combined form.

The scope of the present disclosure is indicated by the following claims rather than the detailed description above, and all changes or modifications derived from the meaning and scope of the claims and equivalent concepts should be construed as being included in the scope of the present disclosure.

## Claims

1. A breast cancer diagnosis system using near-infrared measurement technology, comprising:
a probe that sequentially outputs light of a plurality of wavelengths of a near-infrared area to a target object, receives reflected light reflected from the target object, and sequentially processes the reflected light;
a central control device that controls an operation of the probe, receives optical data of the reflected light sensed by the probe, calculates a concentration of chromophore of the target object for each chromophore based on the optical data, and generates an image of the chromophores indicating a distribution of the concentration value of each chromophore; and
a display that outputs the image generated by the central control device,
wherein in the probe, one or more channel signal processing units including one or more light irradiation modules and one or more light collection modules are disposed, and each of the channel signal processing units sequentially operates so that each of the channel signal processing units generates the optical data of the reflected light.

2. The breast cancer diagnosis system of claim 1, wherein the probe includes
a body that includes the channel signal processing units, a first control unit controlling an operation of the channel signal processing unit, and a first communication unit transmitting output data of the first control unit to an outside of the probe; and
a contact surface that is disposed on a lower surface of the body,
the light irradiation module and the light collection module of the channel signal processing unit are disposed in a state of being exposed to the outside through an opening formed on the contact surface,
the one or more channel signal processing units are disposed in a horizontal direction along one axis of the probe, and
the light irradiation modules and the light collection modules of the channel signal processing unit adjacent to each other are disposed adjacent to each other, the light irradiation modules are disposed in a row parallel to the one axis, and the light collection modules are disposed in a row parallel to the one axis by being spaced apart from the light irradiation modules by predetermined distances.

3. The breast cancer diagnosis system of claim 2, wherein each of the channel signal processing units includes
one or more light irradiation modules disposed adjacent to each other;
the light collection module disposed by being spaced apart from the light irradiation module by predetermined distance; and
a driving chip that sequentially outputs a driving signal for driving the light irradiation module according to a control signal of the first control unit and transmits the optical data of the reflected light detected by the light collection module to the first control unit, and
the driving chip of each of the channel signal processing units sequentially outputs 4 to 12 driving signals so that light of 4 to 12 different wavelengths is sequentially output from the light irradiation module.

4. The breast cancer diagnosis system of claim 2, wherein the first control unit includes
a first decoder that sequentially transmits a control signal for operating a driving chip of each of the channel signal processing units; and
a second decoder that sequentially receives and outputting the optical data of the reflected light output from each of the driving chips.

5. The breast cancer diagnosis system of claim 2, wherein the probe further includes a blinking LED or liquid crystal display in a housing outside the body, and
upon receiving all of the optical data of the reflected light corresponding to a unit scan through each of the channel signal processing units, the first control unit displays that the unit scan is completed through the blinking LED or the liquid crystal display.

6. The breast cancer diagnosis system of claim 1, wherein the central control device calculates the concentration of the chromophore of the target object by inputting a measurement value for each wavelength of the reflected light sensed through the probe to a learning model machine-learned based on training data in which a measurement value for each wavelength of light and the concentration of each of a plurality of chromophore materials are matched respectively.

7. The breast cancer diagnosis system of claim 6, wherein the central control device sequentially receives unit scan data transmitted from each of the channel signal processing units, applies each of the unit scan data to the learning model to calculate the concentration of the chromophore, and displays each concentration of the chromophore on a two-dimensional coordinates to generate a unit frame image is generated,
the unit scan data includes the optical data of the reflected light reflected based on light of 4 or more and 12 or less different wavelengths output from the light irradiation module of each of the channel signal processing units, and
data of each coordinate and an empty space between the coordinates displays an estimated value through an interpolation algorithm.

8. The breast cancer diagnosis system of claim 1, wherein the central control device outputs an examination interface for guiding a scanning procedure through the probe,
the examination interface includes an imaging guide interface displaying a left breast area and a right breast area, respectively, and
a first ROI interface displaying an imaging area requiring diagnosis and a second ROI interface displaying an area requiring imaging as a contrast area for the imaging area requiring the diagnosis are displayed on the imaging guide interface.

9. The breast cancer diagnosis system of claim 8, wherein the examination interface further includes a scanning guide interface indicating a state of securing unit scan data of the probe.

10. The breast cancer diagnosis system of claim 8, wherein the diagnosis interface displays a plurality of ROI interfaces displaying an imaging area requiring diagnosis according to a user's selection, and displays a plurality of ROI interfaces of a contrast area corresponding thereto.

11. The breast cancer diagnosis system of claim 9, wherein the central control device outputs a diagnosis interface that outputs the chromophore image indicating a distribution state of the chromophores, and
the diagnosis interface disposes and displays the chromophore image of a diagnosis area generated based on data collected using the first ROI interface and the chromophore image of a contrast area generated based on data collected using the second ROI interface, side by side.

12. The breast cancer diagnosis system of claim 10, wherein the diagnosis interface disposes and displays the chromophore image of the diagnosis area and the chromophore image of a contrast area side by side for each type of the chromophore.

13. A breast cancer diagnosis method using a breast cancer diagnosis system based on near-infrared measurement technology, the breast cancer diagnosis method comprising:
receiving optical data of reflected light from a target object sensed by a probe;
calculating a concentration of chromophore of the target object for each chromophore based on the optical data;
generating an image of the chromophore indicating a distribution of the calculated concentration values of the chromophores; and
outputting the image of the generated chromophore through a display,
wherein in the step of calculating the concentration of the chromophore, the concentration of the chromophore of the target object is calculated by inputting a measurement value for each wavelength of the reflected light sensed through the probe to a learning model machine-learned based on training data in which a measurement value for each wavelength of light and the concentration of each of a plurality of chromophore materials are matched respectively.

14. The breast cancer diagnosis method of claim 13, further comprising;
outputting an examination interface for guiding a scanning procedure through the probe,
wherein the examination interface includes an imaging guide interface displaying a left breast area and a right breast area, respectively, and
a first ROI interface displaying an imaging area requiring diagnosis and a second ROI interface displaying an area requiring imaging as a contrast area for the imaging area requiring the diagnosis are displayed on the imaging guide interface.

15. The breast cancer diagnosis method of claim 14,
wherein the examination interface further includes a scanning guide interface indicating a state of securing unit scan data of the probe.

16. The breast cancer diagnosis method of claim 14,
wherein the examination interface displays a plurality of ROI interfaces displaying an imaging area requiring diagnosis, and displays a plurality of ROI interfaces of a contrast area corresponding thereto.

17. The breast cancer diagnosis method of claim 13, wherein the step of outputting the chromophore image includes a step of outputting a diagnosis interface that outputs the chromophore image indicating a distribution state of the chromophores, and
the diagnosis interface disposes and displays the chromophore image of a diagnosis area generated based on data collected using a first ROI interface and the chromophore image of a contrast area generated based on data collected using a second ROI interface, side by side.

18. The breast cancer diagnosis method of claim 17, wherein the diagnosis interface disposes and displays the chromophore image of the diagnosis area and the chromophore image of the contrast area side by side for each type of the chromophore.
